# EUROPEAN PATENT SPECIFICATION

(11) **EP 4 182 478 B1**
(45) Date of publication and mention of the grant of the patent: **10.09.2025**
(21) Application number: 21751622.8
(22) Date of filing: 13.07.2021
(51) Int. Cl.: C12Q 1/6883, C12Q 1/70

(54) **DIAGNOSIS IN VITRO OF MULTIPLE SCLEROSIS**
IN VITRO DIAGNOSE VON MULTIPLER SKLEROSE
DIAGNOSTIC IN VITRO DE LA SCLÉROSE EN PLAQUES

(30) Priority: 14.07.2020 IT 202000017113
(43) Date of publication of application: 24.05.2023
(73) Proprietor: Università Degli Studi di Cagliari, 09124 Cagliari (IT)
(72) Inventor: GRANDI, Nicole, 09124 Cagliari (IT); TRAMONTANO, Enzo, 09124 Cagliari (IT); PISANO, Maria Paola, 09124 Cagliari (IT)
(74) Representative: Primiceri, Maria Vittoria
(86) International application number: PCT/IB2021/056306
(87) International publication number: WO 2022/013749

(56) References cited:
- US-A1- 2004 054 133
- GRANDI NICOLE, 5 November 2019 (2019-11-05), Third International Workshop on Human Endogenous Retroviruses and Diseases, pages 1 - 1, XP055781316, Retrieved from the Internet <URL:https://www.hervsanddisease.com/program-2019/> [retrieved on 20210302]
- MORANDI ELENA ET AL: "The association between human endogenous retroviruses and multiple sclerosis: A systematic review and meta-analysis", PLOS ONE, vol. 12, no. 2, 16 February 2017 (2017-02-16), pages e0172415, XP055781339, DOI: 10.1371/journal.pone.0172415
- NOWAK JERZY ET AL: "Multiple Sclerosis-Associated Virus-Related pol Sequences Found Both in Multiple Sclerosis and Healthy Donors are More Frequently Expressed in Multiple Sclerosis Patients", JOURNAL OF NEUROVIROLOGY, vol. 9, no. 1, 1 January 2003 (2003-01-01), GB, pages 112 - 117, XP055781260, ISSN: 1355-0284, DOI: 10.1080/13550280390173355
- VARGIU LAURA ET AL: "Classification and characterization of human endogenous retroviruses; mosaic forms are common", RETROVIROLOGY, vol. 13, no. 1, 1 December 2016 (2016-12-01), pages 1 - 29, XP055781292, DOI: 10.1186/s12977-015-0232-y
- GRANDI NICOLE ET AL: "Contribution of type W human endogenous retroviruses to the human genome: characterization of HERV-W proviral insertions and processed pseudogenes", RETROVIROLOGY, vol. 13, no. 1, 9 September 2016 (2016-09-09), pages 1 - 25, XP055781296, DOI: 10.1186/s12977-016-0301-x

## Description

### Technical field

The present invention relates to a method based on the analysis of the expression of transcripts deriving from Human Endogenous Retrovirus (HERV) for the in vitro diagnosis of Multiple Sclerosis (MS), namely a transcript of SEQ ID NO: 1.

### Prior art

Multiple Sclerosis (MS) is a demyelinating neurodegenerative disease affecting approximately 2.3 million people worldwide. The exact etiology and causes of the disease are still partially unknown, but suggest that the disease originates from a combination of environmental and genetic factors.

To date, there is no single test available that can definitely and indisputably confirm the diagnosis of MS, just as there are no diagnostic or predictive biomarkers for the disease.

Currently, the diagnosis is made by the doctor on the basis of three elements: the symptoms reported by the patient, the neurological examination and the instrumental (magnetic resonance - evoked potentials) and biological (blood and cerebrospinal fluid) analyses. The set of results and prolonged clinical observation allow to confirm or exclude the pathology but, to date, a single test is not available that can definitely and indisputably confirm the diagnosis of MS, just as there are no diagnostic biomarkers or predictive for the disease. Diagnosis is based on parameters affected in part by subjective evaluation (patient-reported symptoms, doctor's evaluation) and in part requiring expensive (magnetic resonance imaging) or invasive practices for the patient (cerebrospinal fluid sampling) to exclude the presence of MS.

Within the human genome are contained sequences that represent remnants of ancient infections by human endogenous retroviruses (HERV). These sequences make up about 8% of our DNA and are highly similar to each other in terms of nucleotide sequence. While the genomic characterization of HERVs is still ongoing, an impressive amount of data has been obtained regarding their general expression in different tissues (Grandi and Tramontano 2017, 2018). Among the HERVs, one of the most studied and most expressed groups is the W group, which has been studied extensively for its putative role in various diseases, such as cancer, inflammation and autoimmunity. Despite the great interest in the link between HERV-W expression and human pathogenesis, no conclusive correlation has so far been demonstrated. In general, (i) the absence of a correct identification of the specific HERV-W sequences expressed in a given condition; and (ii) the lack of studies that try to combine the different observations under the same experimental conditions are the main problems that prevent the definitive evaluation of the impact of HERV-W on human pathophysiology.

Regarding the correlation between HERV and multiple sclerosis, several studies have been proposed over time in which a link between group members and the pathology has been sought. The MSRV (MS-associated retrovirus) presented in Dolei and Perron 2009; H Perron et al. 1997; Voisset et al. 1999 as a "proposed cofactor of multiple sclerosis" has been widely debated in the literature for years, there is currently little evidence not only of its actual involvement, but even of its real existence. It has been proposed as an exogenous member of the HERV-W group but its nature has not been clarified at the moment. MSRV has never been isolated or confirmed as an existing virus. Among the various hypotheses made in this regard, there is also the possibility that it is actually an artifact originated collaterally during HERV study experiments based on sequence amplification processes (Grandi et al., 2016; Laufer et al., 2009). In the past years the same inventors have developed a database characterizing in detail about 3200 HERV sequences integrated into the human genome and classified in a total of 70 taxonomic groups (Vargiu et al. 2016), in this work which focused solely on the classification of these sequences does not refer to the correlation with the pathology of multiple sclerosis. These sequences are present in various positions on all human chromosomes, and their identification and characterization was possible thanks to an innovative software, RetroTector, which scans the genome identifying individual HERV sequences by recognizing conserved retroviral motifs and their relationship within the same integrated sequence (Sperber et al. 2007). The identified sequences are then classified using a multiple approach that includes phylogenetic relationships and structural comparison to a collection of reference retroviral sequences (Jern et al. 2005; Sperber et al. 2007). The dataset obtained uniquely identifies each HERV sequence in terms of genomic coordinates and nucleotide sequence, allowing it to be distinguished from the others and to specifically associate the resulting expression products. Moreover, this dataset, being based on the identification of conserved retroviral domains, includes the most intact HERV sequences and therefore more suitable to be still able to produce expression transcripts. To maximize the level of detection of HERV sequences of possible interest, the starting database was then integrated into a second characterization phase, focused on all members (regardless of the degree of conservation of the individual sequences) of some individual groups. HERV of particular relevance for MS and/or probable transcriptional activity, such as the HERV-W, HERVK (HML10), HERV-K (HML7) and HERV-K (HML6) groups (Grandi et al., 2016, 2017, 2021; Pisano et al., 2019). In particular, the HERV-W group is currently the most studied in relation to MS, with the first studies dating back to the 1990s and reporting the isolation of viral particles attributable to these sequences in MS patients (Grandi and Tramontano 2017). In the following decades, the HERV-W group was repeatedly hypothesized as a probable contributor to the disease, based on the results of multiple studies reporting i) greater expression of the group in biological fluids and brains of MS patients, ii) the presence of HERV-W proteins in the brain of MS patients, especially at the level of active lesions, iii) an immune response of the patient, both in the form of specific antibodies and with non-specific inflammatory phenomena against these proteins (Grandi and Tramontano 2017). In recent years, the HERV-W Envelope protein has shown neuro-inflammatory properties capable of developing cerebral symptoms coinciding with the main characteristics of MS (Van Horssen et al. 2016). The same protein has been studied in mouse models, confirming the ability to induce neuro-inflammation and damage to oligodendrocytes and myelin, resulting in an MS-like allergic encephalomyelitis (Antony et al. 2004; Grandi and Tramontano 2017, 2018; Perron et al. 2013). In light of these findings, a monoclonal antibody against HERV-W Env has shown neutralizing effects in vitro and in MS mouse models, restoring myelin expression, and is currently in phase II of clinical development (Curtin et al. 2015). Despite a clear indication of the pathogenic potential and of its exploitation as a therapeutic target, to date the debated association between HERV-W and MS still lacks both the identification of the specific HERV-W sequences that produce this Env protein, and the characterization of its/their expression in MS patients compared to healthy individuals. As for the other characterized groups, the HERV-K group (HML10) has a localized insertion in the C4 gene of the major histocompatibility complex, determining a polymorphic variant that has been investigated for a possible role in autoimmune diseases, this being the region genomics associated with the largest number of disorders of this type (Grandi et al. 2017; Trowsdale and Knight 2013). The KERV-K group (HML6), on the other hand, is one of the most expressed among all the HERV groups: various transcripts and peptides attributable to it have been reported both in physiological conditions and in various pathologies (Pisano et al. 2019). There are several studies in which attempts have been made to associate HERVs and/or their expression with multiple sclerosis.

To date, however, no HERV group or sequence has been recognized as actually associated with a human disease in general, and more particularly with multiple sclerosis.

It is evident that one of the main problems encountered in the study of the expression of HERV in various pathologies, including sclerosis, is precisely the fact that - being highly identical sequences - the use of generic primers or probes (and therefore not selective for each individual member of the HERV group analyzed) leads on the one hand to amplify only an indiscriminate portion of the sequences of interest, and on the other hand it makes the in vitro formation of chimeric sequences in reality non-existent very likely. Very often then the primers are designed for a single gene of the group, so if that gene is mutated or even is missing due to a deletion (as often happens for these sequences, which have been in the primate genome for millions of years and have accumulated many mutations) a portion of group members will not be detected.

The vast majority of expression studies conducted so far in patients with multiple sclerosis have been based on this approach, in which, through the use of unique primers for an entire HERV group, an increased expression was observed in patients compared to controls, without but to know which specific members of the group were actually expressed. The vast majority of the works cited in (Grandi and Tramontano 2017, 2018; Grandi and Tramontano 2018) belong to this vast group of studies. The results in this sense are largely contradictory, with different works that on the one hand support the increased expression of a group in MS patients and on the other report the absence of differences between cases and controls (Grandi and Tramontano 2017 and 2018). Finally, many of the studies cited are aimed at demonstrating that a HERV group or protein expressed more in patients is involved in the development of the disease, something that has never been confirmed for any pathology so far. The need is therefore felt to investigate the expression of HERV sequences with a different technological approach from that on which traditional HERV expression studies are based, allowing to identify specific HERV sequences of interest in the different groups with high resolution instead of measuring specifically the expression of a set of variously classified elements.

Grandi Nicole: 5 November 2019 (2019-11-05), Third International Workshop on Human Endogenous Retroviruses and Diseases; URL:https://www.hervsanddisease.com/program-2019/ indicates the aim of clarifying the contribution of individual HERV loci to both healthy and MS transcriptome, to identify loci differentially modulated in MS patients. Using RNA-seq from peripheral blood cells, a total of 53 HERVs loci were found to be differentially expressed (DE-HERVs) in MS patients as compared to healthy controls.

MORANDI ELENA ET AL: "The association between human endogenous retroviruses and multiple sclerosis: A systematic review and meta-analysis", PLOS ONE, vol. 12, no. 2, 16 February 2017 (2017-02-16), is a literature review referring to different studies according to which RNA expression of HERVs as detected as being differentially expressed in multiple sclerosis patients as compared to healthy controls. In these studies, the analysis of HERV expression relies on methods that cannot distinguish highly similar HERV sequences, being based on their nucleotide sequence.

In NOWAK JERZY ET AL: "Multiple Sclerosis-Associated Virus-Related pol Sequences Found Both in Multiple Sclerosis and Healthy Donors are More Frequently Expressed in Multiple Sclerosis Patients", JOURNAL OF NEUROVIROLOGY, vol. 9, no. 1, 1 January 2003 (2003-01-01), pages 112-117, GB ISSN: 1355-0284, DOI: 10.1080/13550280390173355, higher frequency of expression of MSRV sequences was detected in lymphocyte RNA as well as in the serum of MS patients in comparison to control groups, including healthy subjects. On the basis of obtained results, NOWAK claims that expression of MSRV RNA is specific for MS. The actual existance of MSRV is debated in literature, since it is claimed as an exogenous counterpart of the HERV-W group that wasbut never isolated, and several studies suggest it could represent an artifact arose from the *in vitro* recombination of highly similar amplicons derived from identical HERV-W group members, confirming the biases associated to this technique when primer specificity is not carefully accounted.

US 2004/054133 discloses a method of diagnosing multiple sclerosis by detection of RNA encoding HERV-W superantigen, e.g. using amplification and sequencing.

In view of the above, the need to identify markers strongly and uniquely correlated to the presence of MS pathology and the need for an analysis method capable of overcoming the technical problems related to the methods commonly used for the analysis of HERV-W sequences remain unsatisfied, i.e. cross-reactivity (amplification of several similar sequences recognized by the same primers), nondetection (non-amplification of mutated sequences at the level of the sites recognized by the primers), and the formation of artifacts.

### Summary of the invention

The problems posed by the known art have been solved with the method according to the present invention.

The invention is set out in the appended set of claims and relates to SEQ ID NO: 1.

The inventors have now devised a method for selecting molecular markers for use in the diagnosis of multiple sclerosis; in particular, the inventors carried out a precise molecular analysis by quantifying the expression levels of HERV transcripts in samples of nucleic acids isolated from blood obtained from patients with multiple sclerosis, comparing said levels with those detected in samples obtained from healthy donors.

The inventors found and selected from a database of about 3200 HERV sequences (HERVdb), 43 sequences with a significant modulation of expression, which was found to be up-regulated or down-regulated, in patients with multiple sclerosis, thus correlating this de-regulation with the presence of the pathology. Finally, the inventors also selected 17 particularly preferred HERV transcripts, with particularly significant levels of deregulation in patients suffering from multiple sclerosis.

Therefore, the subject of the present disclosure are the HERVs shown in tables 2 and 3 and in particular the HERV of SEQ ID NO: 1 for use as biomarker in the in vitro diagnosis of multiple sclerosis.

The present disclosure also relates to a method for the diagnosis of multiple sclerosis, the method comprising the steps of:
- isolate total cellular RNA ex vivo from a sample obtained from an individual
- determine the expression levels of HERV present at the loci indicated in tables 2 and/or 3
- compare said expression levels with the expression levels of the respective HERV detected in a sample obtained from a healthy subject,
in which altered expression levels (both positive and negative) of at least one of said HERV sequences in the sample obtained from the individual compared to the levels detected in a sample derived from a healthy control are indicative of the presence of MS in the subject.

The subject of the present invention is an in vitro method for the diagnosis of multiple sclerosis in an individual, the method comprising the following basic steps:
- to isolate ex vivo an RNA sample from a biological sample obtained from a subject;
- determine the expression levels of the transcript of the HERV sequence SEQ ID No. 1;
- compare these expression levels with the expression levels of the same HERV detected in an RNA sample isolated ex vivo from a sample obtained from a healthy individual in which the decreased expression levels of these HERV in the test sample compared to the levels detected in the sample obtained from a healthy individual are indicative of the presence of multiple sclerosis in the subject.

In a preferred embodiment, the expression analysis step of the HERV transcripts is carried out using next generation sequencing techniques, in particular RNAseq and subsequent bioinformatic analysis of the results.

In particular, the HERV of SEQ ID No 1 is for the use in an in vitro diagnostic method of multiple sclerosis; in particular, the object of the present invention is the HERV of SEQ ID No 1 for use as biomarker in the in vitro diagnosis of multiple sclerosis, in which expression levels decreased by the HERV transcript of SEQ ID NO: 1 compared to the levels found in samples obtained from a healthy individual is indicative of the presence of multiple sclerosis in the subject.

Other objects will become apparent from the detailed description of the invention which follows.

### Brief description of the Figures

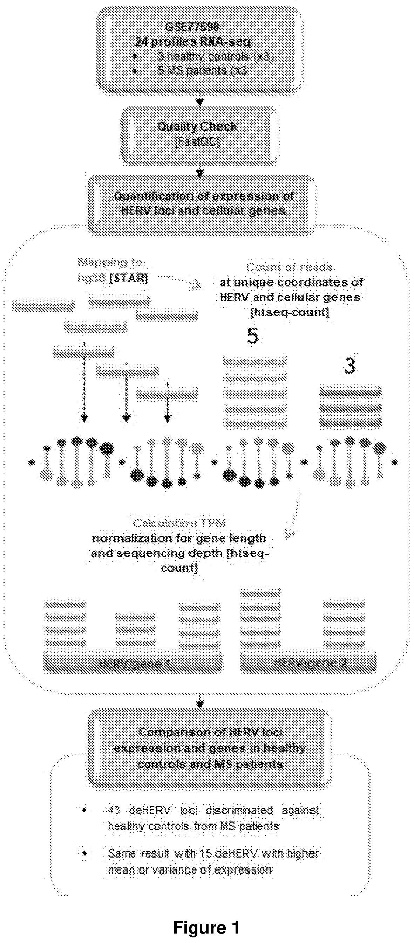

**Figure 1****. Bioinformatics pipeline used for expression analysis**
   The main software and tools used in the various phases are shown in square brackets. MS = multiple sclerosis, HERV = human endogenous retroviruses, hg38 = reference sequence of the human genome GRCh38/hg38, TPM = transcripts per million kilobases, deHERV = differentially expressed HERV sequences in patients with multiple sclerosis.

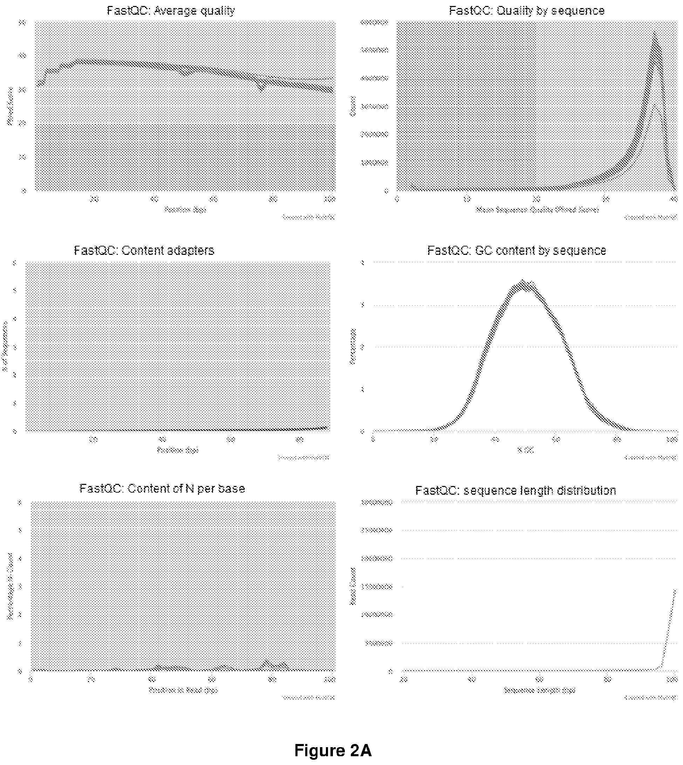

**Figure 2A****. Quality control of the reads contained in the RNAseq GSE77598 dataset**
   Performed using the FastQC software. *Mean quality score* = average quality for each nucleotide position of the reads of each sample, *by sequence quality score* = average quality of the reads of each sample, *adapter contents t=* residual presence of adapters used in the sequencing process, *by sequence GC content =* percentage content of GC bases (expected = 50%), *by base N content =* percentage of unassigned bases for each nucleotide position of the reads of each sample, *sequence length distribution =* distribution of the reads of each sample in terms of length (expected = 100 bp).
**Figure 2B****. Quality control of the mapping of the reads to the reference sequence of the human genome (GRCh38/hg38)**
   Carried out using the FastQC software. *STAR alignment score=* average quality of the alignment of the reads of each sample in terms of the number of uniquely mapped reads. The graph instead shows the relationship between the number of mapped reads and the total number of reads per sample.
**Figure 3****. Principal Component Analysis (PCA) Responsible for Variance in HERV Expression Between Samples**
   The healthy control or multiple sclerosis patient condition is the major variance component of HERV expression in samples (PC1, responsible for 39 % of variance). Note the clear division of healthy controls and patients, indicative of the discriminating power of HERV expression for multiple sclerosis. The samples representing biological triplicates of the same individual are grouped together.

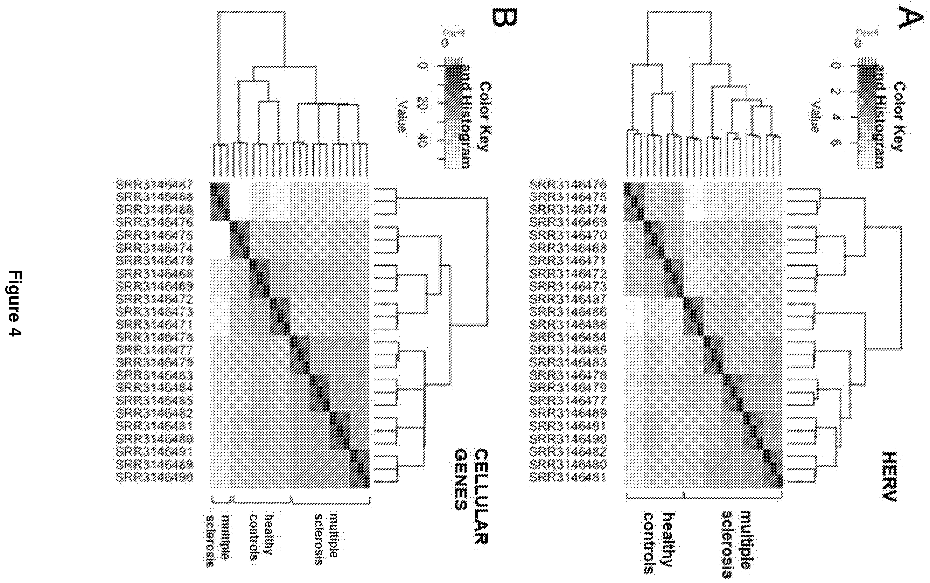

**Figure 4****. Distance between samples based on global expression of HERV (A) and cellular genes (B)**
   Note the clear division of healthy controls and patients based on HERV expression, indicative of the discriminating power for multiple sclerosis. The triplicate representative samples of the same individual are grouped together.

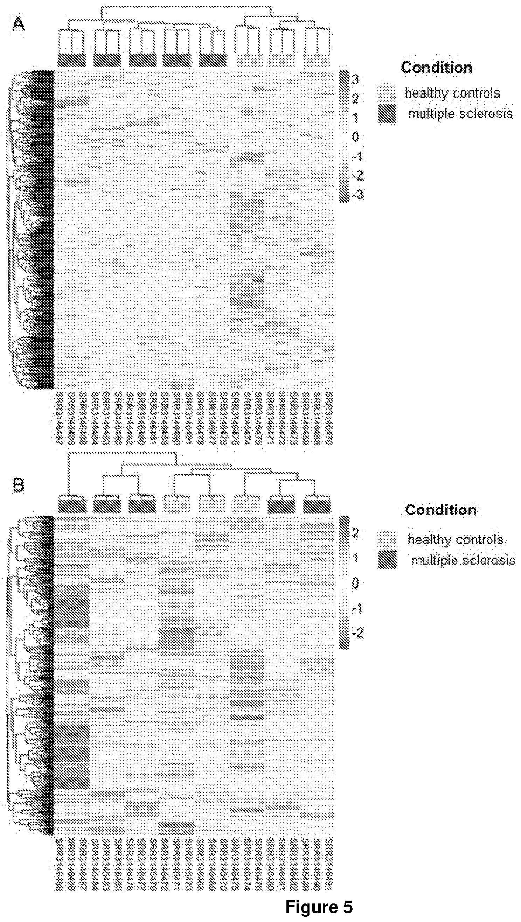

**Figure 5****. Heatmap of the 400 HERV loci (A) and cellular genes (B) with the highest mean expression**
   Note the clear division of healthy controls and patients, indicative of the discriminating power of HERV expression for multiple sclerosis (A). On the other hand, this division is not observable considering the cellular genes (B). The triplicate representative samples of the same individual are grouped together.

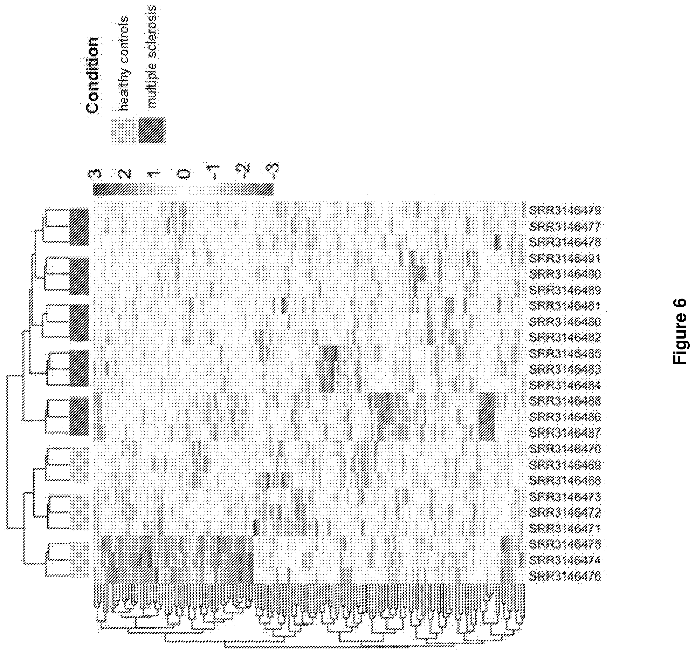

**Figure 6****. Heatmap of 140 HERV loci with the highest mean expression**
   This set of loci, extracted from the larger one represented in Figure 5, represents the minimal set of HERV loci with discriminating power for multiple sclerosis, as indicated by the clear division of healthy controls and patients. The triplicate representative samples of the same individual are grouped together.

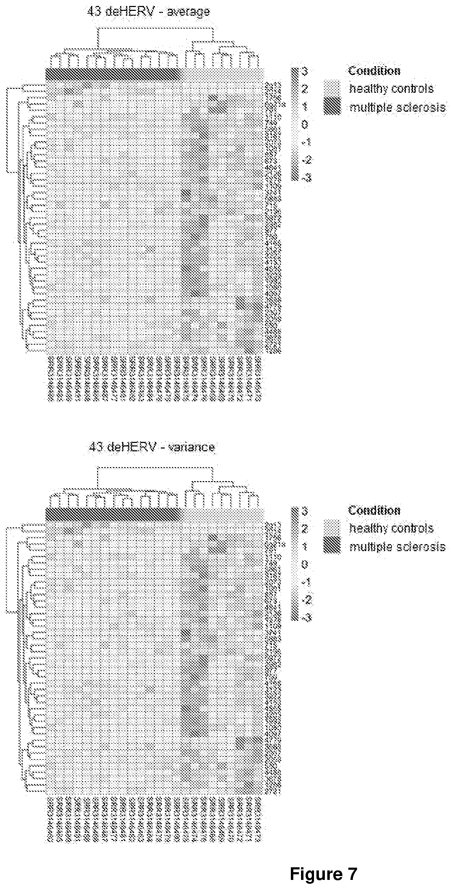

**Figure 7****. Heatmap of 51 deHERV discriminating loci based on mean (A) and variance (B) expression values**
   This set of HERV loci was found to be differentially expressed in the multiple sclerosis condition based on the statistical comparison between the expression levels of the same locus in healthy controls and patients (adjusted p-value ≤ 0.01 - log2 fold change> 1 in case of expression upregulation or <-1 in case of expression downregulation). Note the clear division of healthy controls and patients, indicative of the discriminating power of HERV expression for multiple sclerosis.

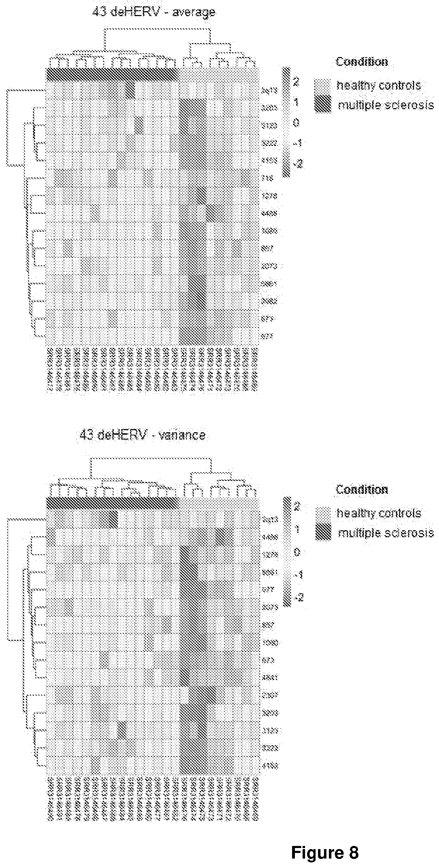

**Figure 8****. Heatmap of the 15 discriminating deHERV loci with highest mean expression (A) and variance (B)**
   This subset of deHERV loci, extracted from the larger one represented in Figure 7, represents the minimum set of deHERV loci with discriminating power for the multiple sclerosis, as indicated by the clear division of healthy controls and patients.

### Detailed description

The following definitions are given within the scope of the present disclosure.

*By primer* we mean an oligonucleotide of variable length which acts as a starting point for the synthesis of a nucleic acid of interest, which is recognized by sequence complementarity, allowing its selective amplification by means of a PCR (Polymerase Chain Reaction) reaction.

*A probe* is defined as an oligonucleotide of variable length that hybridizes to a complementary target nucleic acid.

For *NGS (Next Generation Sequencing)* or *Deep sequencing* is a set of technologies that allow to sequence within a narrow time entire genomes or transcriptomes

For *RNA-Seq* means the application of a method to NGS sequencing of an RNA sample

For *transcriptome* is means the sequencing product of all the transcripts present in a certain cell or tissue population, using NGS techniques. Within the scope of the present invention, transcriptome sequencing was performed specifically on the polyadenylated portion of the transcripts (corresponding to messenger RNA or mRNA).

By *"reads"* we mean the sequences originating from the sequencing process, which represent the fragments of cellular transcripts and will be present in quantities directly proportional to the level of expression of the gene that originated them. Reads can be of variable length, and be single or paired at one end.

By *mapping* we mean the alignment of the transcriptome reads to a reference genomic sequence, in order to identify its genetic origin. This process is implemented using bioinformatics software and involves the elimination of reads that do not map to a unique genomic location.

For *expression analysis quantitative* means the bioinformatic quantification of the expression of a given gene or genomic element by counting the map that reads at its genomic coordinates and normalization based on factors such as the length of the gene itself and the sequencing depth of the transcriptome. In this way, an expression value quantified in Transcripts Per Million Kilobases (TPM) is obtained, which allows the comparison of the expression of different genes.

By d*ifferential expression analysis* we mean the statistical comparison of the expression levels of specific cellular genes or HERV, to identify significantly modulated elements (over-expressed or under-expressed) in a certain condition (in this case, MS).

By *HERV locus* we mean the single insertions of endogenous retroviruses present in the human genome (plural = loci).

The inventors with the method of the invention described below have overcome the technical problems relating to the traditional analysis of HERV sequences and their expression caused by the use of primers and probes, i.e. cross reactivity (amplification of several similar sequences recognized by the same primers) and the lack of detection (lack of amplification of mutated sequences at the level of the sites recognized by the primers), which together with the frequent formation of recombinant artifacts makes it difficult to identify which specific HERV sequence is actually expressed in a certain condition. Using the method described below, the inventors were able to analyze the expression of a large number of HERV sequences in healthy subjects and patients with multiple sclerosis (MS) and were able to arrive at a selection, establishing a clear correlation between the expression of specific HERV sequences present in the human genome and the presence of multiple sclerosis (MS). In particular, the inventors starting from a database (HERVdb) containing about 3200 sequences attributable to the related HERV genomic characterization publications of the Proponents (Grandi et al. 2016, 2017; Pisano et al. 2019; Vargiu et al. 2016) they first identified a set of 43 significant sequences and from these they selected a group of 17 sequences whose most variable expression was found to be predictive of the presence of the disease, allowing to clearly distinguish healthy individuals from MS patients.

The inventors have developed a method capable of obtaining a precise analysis of the expression of HERV transcripts, overcoming the problems of the known art and satisfying the need to detect each specific HERV expressed using Next Generation Sequencing (NGS) techniques and using universal adapters for amplify all polyadenylated cell transcripts, which are then uniquely mapped by bioinformatic alignment of each read to the human genome sequence.

In this way, the specific quantification of the expression of each cellular gene and HERV sequence is based on the quantification of the reads at the level of their unique position in the genome, and is therefore not susceptible to cross-reactivity or ambiguous results. In the specific case of HERVs, the use of sequencing technical characteristics such as paired reads with a length greater than 75 bases each guarantee a high mapping specificity even of highly similar sequences, since they will most likely include a portion of unique sequence. Another guarantee of specificity is that reads that do not uniquely map a specific position in the genome are excluded from the quantitative analysis, avoiding erroneous estimates.

To achieve this sensitivity of discrimination, it is first of all necessary to know the precise position in the genome of the elements to be analyzed.

In particular, the inventors used data derived from NGS experiments in which the transcriptome of healthy individuals and individuals with multiple sclerosis was sequenced.

HERVdb was then used as a starting point to analyze the expression of these elements in the transcriptome of monocytes from peripheral blood of healthy controls (3) and MS patients (5) sequenced in triplicate (24 total samples, contained in a public record registered in the GEO repository (https://www.ncbi.nlm.nih.gov/geo/, accession number: GSE77598). The comparison of the expression levels of each HERV between the two conditions allowed to identify specific sequences that showed a differential expression between healthy controls and MS patients. The methodology used to conduct the analysis is based on the use of an optimized bioinformatics process for the identification, mapping and quantitative evaluation of the transcripts produced by the HERV loci included in the said HERVdb, so compararne statistically presence and abundance in the two conditions taken into consideration (healthy controls and patients with MS).

This process allowed a first phase, to analyze the global expression of HERV loci by selecting the 400 most expressed loci and obtaining a general overview of the expression of these transcripts in healthy and affected individuals (Figure 5A). The inventors then deepened the analysis of the data obtained by considering the transcripts that had the highest variance and standard deviation, as well as reducing the number of HERV loci considered, down to a minimum discriminant set of 140 sequences (Listed in Table 1). The set of 140 HERV loci described in Table 1 is endowed with discriminating power on the basis of the overall expression in the samples under analysis selected on the basis of the expression mean. In order to obtain more effective diagnostic markers, the inventors carried out a further selection step by searching for transcripts with a significant difference in expression between healthy individuals and MS patients. A differential expression analysis was therefore conducted verifying which HERV loci contained in the HERVdb - beyond their expression values - showed a statistically significant variation between healthy controls and MS patients.

The expression variation of the HERV loci between healthy controls and MS patients was measured and expressed in log2 fold change. The HERV loci whose variation between controls and patients reached a threshold of statistical significance, represented by an adjusted p-value (p-adj, according to Benjamini-Hochberg) ≤ 0.01 and by a expression variation between healthy individuals and individuals with MS measured in terms of log2 fold change; loci with log2 fold change value> 1 (in case of expression up-regulation) or <-1 (in case of expression down-regulation) were selected as significant. The HERV loci considered for their discriminating power have an average length of about 8000 nucleotides.

The differential expression analysis allowed to identify 43 HERV loci located on autosomal chromosomes and reported in table 2 which are differentially expressed (deHERV) in healthy and MS patients. Of these 43 HERVs, 41 are down-regulated in patients with multiple sclerosis and only 2 are up-regulated (N ° 4 and 29).

Starting from this set, the inventors finally selected an even smaller number of deHERV loci with the highest discriminating power, identifying as the final subset a total of 17 deHERV loci, ordered on the basis of decreasing mean and variance values of expression, in order to select the most significant ones (Figure 8). These 17 HERVs make it possible to clearly distinguish MS patients from healthy controls (Figure 8, Table 3). The deHERV loci included in this minimal subset have statistical significance levels between 7.08-14 and 0.005, and most loci are down-regulated, with a log2 fold change value between -1.75 and -1.01 in MS patients, except two up-regulated loci with log2 fold change of 1.86 and 2.62.

The inventors have therefore selected, starting from a large initial dataset, HERV sequences with a diagnostic and predictive character of multiple sclerosis. The inventors therefore surprisingly found that the analysis of the expression of HERV present in the initial dataset and selected for their mean or variance of expression in patients has the value of a highly diagnostic and predictive biomarker of multiple sclerosis. In particular, the inventors selected 43 HERV transcripts whose differential expression in healthy subjects and MS patients has a high diagnostic power and, among these, 17 particularly preferred transcripts for the diagnosis of the pathology.

The inventors have developed a method for the ex vivo diagnosis of this pathology which includes the following basic steps
- isolate total cellular RNA ex vivo from a biological sample obtained from an individual;
- determine the expression levels of HERV present at the loci indicated in tables 2 and/or 3;
- compare said expression levels with the expression levels of the respective HERVs detected in a biological sample obtained from a healthy subject,
in which decreased expression levels of said sequence HERV in the sample obtained from the individual versus the levels found in a sample derived from a healthy control are indicative of the presence of MS in the subject.

In a preferred embodiment, the method according to the disclosure comprises the steps of:
- isolate total cellular RNA ex vivo from a biological sample obtained from an individual;
- selection and deep sequencing of polyadenylated RNA to generate sequencing reads and subsequent bioinformatic analysis performed through a computer system.
Said analysis comprising
- align the sequencing reads obtained with a reference human genome;
- determining relative quantities of the reads corresponding to the sequences reported in table 2 and/or preferably in table 3;
- statistically compare said relative quantities with the relative quantities detected in a sample obtained from a healthy subject, preferably by calculating the Fold change on a logarithmic scale and the adjusted p value and in which they are selected as significant with an adjusted p value of ≤ 0.01 and log2 fold change> 1 (in case of expression up-regulation) or <-1 (in case of expression down-regulation).
in which altered expression levels (both positive and negative) of at least one of said HERV sequences in the sample obtained from the individual compared to the levels detected in a sample derived from a healthy control are indicative of the presence of MS in the subject.

The results obtained using the method according to the invention are intended to be submitted to the attending physician for subsequent and appropriate diagnostic investigations in order to confirm the diagnosis and identify suitable treatments.

In detail, the inventors have used a method and a bioinformatic process represented in Figure 1 and schematized in the following phases:

### · Step 0: RNAseq Dataset Download and Quality Control

The GSE77598 dataset was downloaded from NCBI's Gene Expression Omnibus (GEO) (https://www.ncbi.nlm.nih.gov/geo/) in the form of raw transcriptomic data, ie including the pairs of reads of each sample (read_1 and read_2) in two files of type fastq. The raw data was then subjected to Fastqc software quality control (https://www.bioinformatics.babraham.ac.uk/projects/fastqc/) to evaluate parameters related to the sequencing process, including length and total number of reads, nucleotide content, presence of ambiguous bases, content of GC bases and residual presence of adapters used for sequencing. The overall quality was satisfactory (Figure 2A), allowing the use of the dataset for the subsequent phases.

### · Phase 1: Mapping the reads to the reference human genome

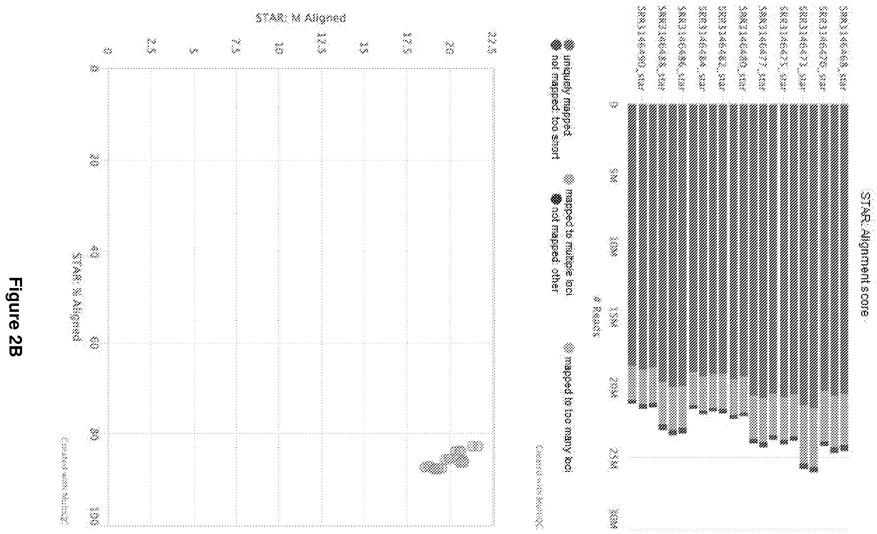

The reads_1 and _2 of each sample were aligned to the most up-to-date reference sequence of the human genome currently available (GRCh38/hg 38) downloaded as a GTF file from NCBI Genome Browser (https://hgdownload.soe.ucsc.edu/downloads) using the STAR software, and all unmapped or non-uniquely mapped reads (therefore aligned in two or more genomic locations) have been eliminated. Even the obtained alignments were checked in terms of quality before proceeding to the subsequent stages, showing a good percentage of reads mapped uniquely (on average 86%) (Figure 2B)

### · Phase 2: quantification of the expression of each HERV and cellular gene

The alignments carried out were used to count the reads aligned to each of the genomic coordinates uniquely identifying the HERV loci (HERVdb) and cellular genes (Gencode, version 29) (Harrow et al. 2012) using the HtseqCount software (Anders, Pyl, and Huber 2015). This allowed to obtain the raw number of counts of the reads uniquely mapped to each HERV/gene. Furthermore, in order to have comparable values, the expression levels of each HERV/gene were also calculated in terms of Transcripts Per Million kilobases (TPM), by importing the raw counts on the Rstudio (Rstudio Team 2016) platform. normalization based on factors such as HERV/gene length and transcriptome sequencing depth.

### · Phase 3: analysis of the variability of the expression of HERV and cellular genes in MS patients compared to healthy controls

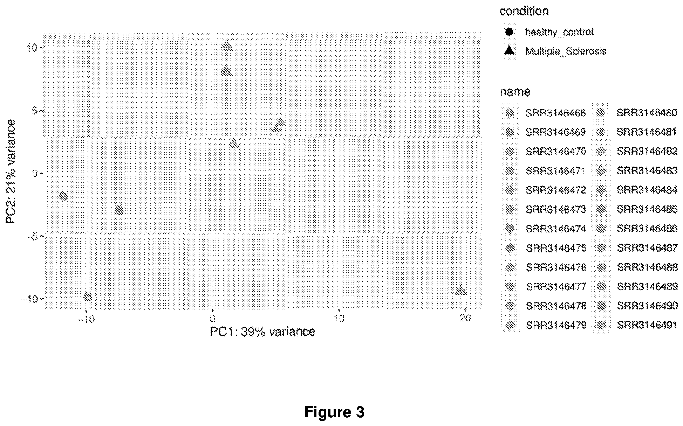

The raw results obtained from the count of the reads mapped in each HERV locus and cellular gene were imported into the Rstudio platform (Rstudio Team 2016) and subjected to Rlog normalization (regularized-logarithm transformation, transforms the raw counts into log2 logarithmic scale, minimizing the differences between samples and with respect to the size of the dataset). The first analysis carried out was to identify the main components (PC) determining the variability in the expression of HERV and genes among all the samples. This analysis was carried out using unsupervised PCA (Principal Component Analysis), i.e. without indicating a particular condition of interest but evaluating the PCs based on the distribution of the samples according to their variance values in the expression of HERV and cellular genes. The result of the PCA (Figure 3) identified as PC1 (ie the main variance component) the status of "healthy" or "affected by MS". In fact, this condition alone is responsible for 39% of variance, and determines the net division of the samples by PC1 into two distinct groups, which correspond precisely to controls and patients (Figure 3). Confirming the quality of the dataset and the identifying power of the pipeline, the samples derived from the triplicate sequencing of the same individual in turn form well appreciable subgroups, reflecting an inter-individual variability that in any case does not influence the clear discrimination of patients and controls.

In light of this characteristic global expression difference in the presence of MS, the variability of expression of individual HERV and cellular genes between MS patients and healthy controls was compared both in terms of distance between samples (Figure 4) and in terms of mean expression (Figure 5). Both analyses confirmed that the global variation of HERV expression has a discriminating power for the condition of MS, allowing to clearly divide the sick individuals from the healthy controls into two well-defined groups, including in turn the subgroups with the 3 replicates of sequencing of the same individual (Figures 4A and 5A). On the contrary, the same analysis conducted on the expression of cellular genes did not show any discriminating power (Figures 4B and 5B).

### · Phase 4: Analysis of HERV Loci Expression in MS Patients and Healthy Controls

The discriminating power of HERVs for the MS condition was initially observed by considering the global expression of the HERV loci (as depicted in Figure 5A for the 400 HERV loci with the highest expression mean in the analyzed samples). The same results were also obtained by conducting the analysis based on the highest variance and standard deviation, as well as reducing the number of HERV loci considered to a minimum discriminant set of 140 sequences (Table 1). These sequences, although overall able to divide healthy controls from MS patients, do not, however, have a statistically significant variation in expression between the two groups in all cases.

**Table 1. Description of the set of 140 HERV loci discriminating for MS**

| **ID HERV** | **Coordinates hg38 (chromosome: beginning-end)** | **Bases** | **St.** | **Group** | **Avera ge TPM (C)** | **Averag e TPM (SM)** |
|---|---|---|---|---|---|---|
| 1114 | 3:129168485-129171834 | 3350 | - | ERRANTI-like | 632.64 | 729.43 |
| 6095 | 1:169683482-169691301 | 7820 | + | HERVH | 174.03 | 189.55 |
| 4713 | 19:36149712-36161023 | 11312 | - | HERVH | 102.64 | 114.80 |
| 4720 | 19:38823108-38837433 | 14326 | + | HERV9 | 36.60 | 36.60 |
| 3656 | 11:121632566-121643491 | 10926 | - | HERVH | 28.61 | 25.77 |
| 6171 | 1:207632285-207641252 | 8968 | - | HML2 | 23.48 | 26.54 |
| 4796 | 19:58305729-58315116 | 9388 | + | HML6 | 19.96 | 25.28 |
| 2453 | 7:30572445-30579657 | 7213 | - | HERV4 | 24.63 | 29.43 |
| 6062 | 1:150628159-150635776 | 7618 | - | HML2 | 18.94 | 19.00 |
| 4184 | 14:73702886-73716167 | 13282 | + | HERVH | 10.15 | 10.12 |
| 1658 | 4:153690317-153693920 | 3604 | - | HML3 | 28.64 | 38.76 |
| 3776 | 12:42440799-42451511 | 10713 | + | HML5 | 8.77 | 11.68 |
| 4849 | 20:49281128-49287343 | 6216 | - | HERVIP | 17.34 | 17.06 |
| 6069 | 1:155626674-155635703 | 9030 | - | HML2 | 8.67 | 12.96 |
| 4378 | 16:35797699-35804523 | 6825 | + | HML5 | 13.91 | 16.32 |
| 4331 | 16:2660502-2669985 | 9484 | - | HML4 | 7.81 | 10.29 |
| 3927 | 13:19626060-19635907 | 9848 | + | HML3 | 7.80 | 8.36 |
| 4618 | 19:11942587-11948985 | 6399 | - | HERVE | 10.50 | 11.32 |
| 2521 | 7:64990455-64999936 | 9482 | - | HERV3 | 6.81 | 7.77 |
| 510 | 2:37124923-37137627 | 12705 | + | HERVFA | 3.49 | 7.88 |
| 4185 | 14:75577964-75587648 | 9685 | - | HERVH | 6.03 | 7.54 |
| 699 | 2:142929044-142937291 | 8248 | - | HERVH48 | 8.86 | 7.43 |
| 1884 | 5:76881216-76889560 | 8345 | - | HERVH | 8.26 | 5.07 |
| 5108 | Y:13282562-13289605 | 7044 | + | HARLEQUIN | 6.74 | 7.53 |
| 4778 | 19:53433375-53443110 | 9736 | - | HERV3 | 5.10 | 4.98 |
| 2637 | 7:135174033-135184466 | 10434 | + | HERVIP | 4.12 | 5.10 |
| 1045 | 3:101692114-101701253 | 9140 | + | HML2 | 4.75 | 5.66 |
| 3317 | 10:89406288-89417471 | 11184 | + | HERV9 | 2.61 | 6.62 |
| 6283 | 22:39052278-39066270 | 13993 | - | HERVH | 2.76 | 3.80 |
| 3511 | 11:60215182-60225509 | 10328 | + | HERVL | 3.83 | 4.99 |
| 864 | 3:9841750-9854710 | 12961 | - | HML2 | 3.18 | 3.44 |
| 2384 | 6:158611703-158621178 | 9476 | - | HERVFA | 4.70 | 4.37 |
| 6074 | 1:156180078-156187897 | 7820 | - | HML5 | 6.02 | 5.73 |
| 4329 | 15:101072781-101079317 | 6537 | + | HERVH | 6.49 | 6.17 |
| 3701 | 12:9959546-9968656 | 9111 | - | HERVH | 3.64 | 6.23 |
| 2030 | 5:160116064-160131138 | 15075 | + | MLT | 2.47 | 2.55 |
| 5429 | X:63426518-63434693 | 8176 | - | LTR46 | 4.17 | 4.23 |
| 4758 | 19:51976955-51992228 | 15274 | + | HML6 | 2.29 | 2.16 |
| 6262 | 22:16611312-16616782 | 5471 | + | HERVH | 6.37 | 7.16 |
| 4353 | 16:21629614-21637525 | 7912 | - | HERV9 | 4.50 | 4.14 |
| 3547 | 11:74869434-74884487 | 15054 | + | HUERSP3 | 2.26 | 1.96 |
| 6228 | 1:235670261-235675550 | 5290 | + | HERVIP | 6.63 | 5.25 |
| 673 | 2:127369875-127377853 | 7979 | + | HML5 | 5.57 | 2.89 |
| 2476 | 7:43853008-43866752 | 13745 | - | HML3 | 1.99 | 2.27 |
| 4795 | 19:57896518-57903857 | 7340 | + | HML1 | 3.93 | 4.15 |
| 3949 | 13:40874210-40881161 | 6952 | - | HERVE | 4.31 | 4.05 |
| 4490 | 17:80537010-80552020 | 15011 | - | HML2 | 1.86 | 1.92 |
| 2534 | 7:77339937-77345880 | 5944 | + | HERVH | 6.50 | 3.81 |
| 3902 | 12:111816238-111825646 | 9409 | - | HML6 | 4.22 | 2.38 |
| 2382 | 6:158156792-158169360 | 12569 | - | unclassifiable | 2.18 | 2.30 |
| 624 | 2:97821110-97829456 | 8347 | + | HERV9 | 3.73 | 3.04 |
| 1892 | 5:82267546-82273706 | 6161 | - | HARLEQUIN | 5.10 | 3.96 |
| 4766 | 19:52649616-52653116 | 3501 | + | HERVIP | 9.74 | 6.44 |
| 2776 | 8:41590933-41598326 | 7394 | - | HERVE | 4.11 | 3.23 |
| 3107 | 9:92071888-92078398 | 6511 | + | HERVH | 4.85 | 3.50 |
| 570 | 2:71388088-71395557 | 7470 | + | HML5 | 3.52 | 3.44 |
| 4485 | 17:75252632-75258297 | 5666 | + | HERVH | 4.97 | 4.28 |
| 2152 | 6:57069287-57080996 | 11710 | + | HML5 | 2.20 | 2.15 |
| 6072 | 1:155650288-155659631 | 9344 | - | HERV4 | 2.41 | 2.84 |
| 4790 | 19:57487177-57500813 | 13637 | + | unclassifiable | 1.56 | 1.99 |
| 4332 | 16:3079068-3081318 | 2251 | - | HML3 | 8.73 | 12.52 |
| 558 | 2:64252675-64258565 | 5891 | + | HERVH | 3.46 | 4.51 |
| 3222 | 10:35403060-35410120 | 7061 | - | HERVH | 5.23 | 2.54 |
| 3687 | 12:8281117-8291582 | 10466 | - | HERVE | 2.14 | 2.38 |
| 4744 | 19:47047398-47055397 | 8000 | - | HERVH | 3.56 | 2.60 |
| 4335 | 16:8837971-8845472 | 7502 | - | HERVH | 3.50 | 3.00 |
| 3403 | 11:10004937-10020706 | 15770 | + | MER50 | 1.82 | 1.17 |
| 4050 | 13:99488624-99496752 | 8129 | - | HERV9 | 2.07 | 3.26 |
| 4352 | 16:20674233-20681995 | 7763 | + | HERVI | 2.51 | 3.06 |
| 1885 | 5:76909228-76912976 | 3749 | + | HERV9 | 6.78 | 5.35 |
| 4003 | 13:76983510-76993630 | 10121 | + | MER4 | 2.05 | 2.25 |
| 3409 | 11:17370180-17379293 | 9114 | + | HUERSP3 | 2.48 | 2.31 |
| 909 | 3:32458604-32467714 | 9111 | - | HERVH | 2.88 | 2.12 |
| 2p16.2 | 2:53756639-53761426 | 4788 | + | HERVW | 3.15 | 5.65 |
| 3170 | 9:133031013-133037385 | 6373 | - | HERVIP | 3.19 | 3.20 |
| 3370 | 10:120844913-120851580 | 6668 | + | HERVH | 2.45 | 3.43 |
| 1495 | 4:88442633-88453881 | 11249 | + | HERV4 | 1.11 | 2.58 |
| 934 | 3:44329728-44340878 | 11151 | - | MER84 | 1.64 | 1.91 |
| 2518 | 7:64679995-64686561 | 6567 | + | HERVH | 2.60 | 3.42 |
| 1745 | 5:1577341-1585389 | 8049 | - | HML3 | 1.46 | 3.60 |
| 4426 | 17:11971744-11978102 | 6359 | + | HERVH | 2.35 | 3.11 |
| 4611 | 19:9626252-9638082 | 11831 | + | HERVIP | 1.53 | 1.41 |
| 7p14.2 | 7:35696138-35697017 | 880 | + | HERVW | 17.86 | 20.72 |
| 4215 | 14:92622417-92629608 | 7192 | - | HERVIP | 3.06 | 1.89 |
| 2984 | 8:145055669-145066696 | 11028 | + | HML4 | 1.41 | 1.53 |
| 4613 | 19:9741762-9751748 | 9987 | + | HERVH | 2.11 | 1.39 |
| 4765 | 19:52620842-52627302 | 6461 | + | HERVH | 2.62 | 2.38 |
| 5047 | Y:7377455-7388167 | 10713 | - | HERV9 | 6.54 | 0.02 |
| 3203 | 10:20118010-20124934 | 6925 | - | HERVH | 3.34 | 1.71 |
| 3600 | 11:94650031-94657830 | 7800 | + | HERV4 | 1.77 | 2.02 |
| 3436 | 11:34897254-34907021 | 9768 | + | HUERSP3 | 1.90 | 1.42 |
| 4736 | 19:43321084-43329393 | 8310 | - | HERVH | 1.05 | 3.54 |
| 3901 | 12:110403279-110411068 | 7790 | - | HERVH | 1.79 | 1.88 |
| 2909 | 8:97175022-97180753 | 5732 | + | HERVH | 2.90 | 2.33 |
| 857 | 3:5140823-5149818 | 8996 | - | HERVADP | 2.04 | 1.14 |
| 1112 | 3:128919296-128929231 | 9936 | + | HML1 | 1.61 | 1.13 |
| 1278 | 3:193599956-193613333 | 13378 | - | HEPSI1 | 1.35 | 0.76 |
| 4767 | 19:52627908-52636164 | 8257 | + | HERVH | 2.08 | 1.27 |
| 6245 | 1:246634727-246642803 | 8077 | - | HERVH48 | 1.52 | 1.57 |
| 4153 | 14:53128745-53135507 | 6763 | + | HERVH | 2.90 | 1.26 |
| 6123 | 1:183613210-183622443 | 9234 | + | HERVH | 1.25 | 1.31 |
| 4610 | 19:9634663-9641681 | 7019 | - | HERVH | 1.70 | 1.60 |
| 4147 | 14:50893357-50901034 | 7678 | + | HERVH | 1.96 | 1.24 |
| 493 | 2:26297921-26306401 | 8481 | + | HERVI | 1.04 | 1.53 |
| 838 | 2:233213615-233217829 | 4215 | - | HERVH | 3.49 | 2.17 |
| 3397 | 11:7513515-7520474 | 6960 | - | HERVH | 1.64 | 1.54 |
| 3955 | 13:48056441-48062297 | 5857 | + | HERVH | 2.19 | 1.70 |
| 3387 | 11:4454469-4464776 | 10308 | + | HERVIP | 1.28 | 0,91 |
| 977 | 3:72023117-72028917 | 5801 | + | HERVH | 2.90 | 1,31 |
| 4678 | 19:23340355-23353938 | 13584 | + | HERVH | 0.91 | 0,67 |
| 933 | 3:44340864-44350393 | 9530 | - | HERVH | 1.12 | 1,07 |
| 4488 | 17:77167827-77175201 | 7375 | - | HERVIP | 2.06 | 0.93 |
| 1407 | 4:54020048-54025559 | 5512 | + | HERVH | 2.26 | 1.50 |
| 2124 | 6:35561857-35567823 | 5967 | - | HERVIP | 2.09 | 1.35 |
| 4777 | 19:53469501-53482212 | 12712 | - | HERV3 | 0.72 | 0.79 |
| 3943 | 13:36316325-36321264 | 4940 | + | HERVH | 1.78 | 2.03 |
| 6056 | 1:143764840-143778331 | 13492 | - | MER66 | 0.57 | 0.81 |
| 4770 | 19:52898515-52905434 | 6920 | - | HERVE | 1.23 | 1.47 |
| 6215 | 1:228947563-228951891 | 4329 | + | HERVH | 2.16 | 2.15 |
| 5861 | 1:37907865-37914094 | 6230 | + | HERVH | 2.11 | 1.16 |
| 4763 | 19:52408081-52414733 | 6653 | - | HML6 | 1.26 | 1.50 |
| 4906 | 21:42916753-42925759 | 9007 | - | HERVH48 | 1.37 | 0.85 |
| 1080 | 3:119978070-119990309 | 12240 | + | LTR19 | 1.09 | 0.55 |
| 836 | 2:231091066-231101400 | 10335 | - | HML5 | 1.15 | 0.72 |
| 1638 | 4:139442392-139449817 | 7426 | + | HERVL | 1.26 | 1.23 |
| 4086 | 14:24009540-24015782 | 6243 | - | HML2 | 1.20 | 1.65 |
| 4593 | 19:5158650-5165741 | 7092 | - | HERV9 | 1.59 | 1.04 |
| 546 | 2:58113432-58119130 | 5699 | - | HERVH | 1.63 | 1.52 |
| 6079 | 1:160690812-160699987 | 9176 | + | HML2 | 0.75 | 1.22 |
| 2677 | 7:152027505-152035945 | 8441 | - | HERV9 | 1.22 | 0.92 |
| 2916 | 8:102979373-102991718 | 12346 | - | HEPSI1 | 0.88 | 0.60 |
| 2q22.2 | 2:142898679-142907899 | 9221 | - | HERVW | 1.20 | 0.78 |
| 2574 | 7:97948903-97960343 | 11441 | - | HERVE | 0.84 | 0.71 |
| 466 | 2:6838342-6846883 | 8542 | - | HUERSP3 | 0.37 | 1.92 |
| 4760 | 19:52300607-52307977 | 7371 | - | HML3 | 1.44 | 1.00 |
| 2449 | 7:29636375-29646231 | 9857 | - | HERVT | 0.76 | 0.94 |
| 3123 | 9:100139459-100146544 | 7086 | - | HERVH | 1.66 | 0.88 |
| 2q11.2 | 2:96217144-96223325 | 6182 | - | HERVW | 1.58 | 1.19 |
| 2073 | 6:11103051-11112191 | 9141 | - | HERVFRD | 1.38 | 0.62 |
| 4213 | 14:92041755-92049863 | 8109 | - | HERVH | 1.00 | 1.02 |

| | | | | | | |
|---|---|---|---|---|---|---|
| *ID HERV from Vargiu et al. 2016 and Grandi et al. 2016 (HERV-W). St = strand, TPM = transcripts per million kilobases, C = healthy controls, MS = patients with multiple sclerosis* | | | | | | |

### · Phase 5: Identification and selection of a set of deHERV loci having discriminating power for MS

Although the set of 140 HERV loci described above (Table 1) was endowed with discriminating power based on the global expression in the samples being analyzed, this number of sequences is however quite high, making the potential for variability in the population large. Furthermore, the sequences are selected based on the mean of expression, and not by a significant difference of the latter in patients with MS. Then, in order to select the HERV sequences used for discrimination, a differential expression analysis was performed verifying which HERV loci contained in the HERVdb - beyond their expression values - showed a statistically significant variation between healthy controls and MS patients. Differential expression analysis was performed on the RStudio platform using the DEseq2 package (Love, Huber, and Anders 2014), which estimates variance from raw counts and evaluates differential expression using a negative binomial distribution model. In particular, the variation of expression of the HERV loci between healthy controls and MS patients was measured on a logarithmic scale (log2 fold changes: for example, a log2 fold change of 1.5 corresponds to an increase in expression of a multiplication factor of 2^{1.5}, then to ≈ 2.82 times). HERV loci were then identified as being differentially expressed whose variation between controls and patients reached a threshold of statistical significance, represented by an adjusted p-value (p-adj, according to Benjamini-Hochberg) ≤ 0.01 (the use of the p-adj with respect to the unadjusted p-value decreases the false discovery rate value to 1%) and by a variation of expression between healthy individuals and individuals with MS measured in terms of log2 fold change> 1 (in case of expression up-regulation) or <-1 (in case of expression down-regulation).

Differential expression analysis allowed the identification of 43 differentially expressed HERV loci (deHERV) located on autosomal chromosomes that were evaluated for their discriminating power based on both mean expression and variance, confirming the ability to clearly divide patients with MS from healthy controls (Figure 7). The deHERV loci identified are shown in Table 2 together with the significance level of differential expression in MS (padj) and the TPM values (Transcribed per Million Kilobases, is a quantitative measure of the expression level) mean in healthy controls and patients with SM. The sequences of the HERVs indicated in the table are understood to be incorporated thanks to the reference, present in the table itself, of their exact genomic position, which also specifies the beginning and the end of the sequence on the reference chromosome. The deHERV loci have levels of statistical significance between 7:08⁻¹⁴ and 0.008, and most of the loci is down-regulated in patients with MS (healthy controls: TPM values between 5.6 and 0.1, average = 1.3, median = 0.95 ; MS patients: TPM values between 2.9 and 0.04, mean = 0.7, median = 0.5) (Table 1). The deHERV loci considered for their discriminating power have an average length of about 8000 nucleotides (from a minimum of about 3350 nucleotides to a maximum of about 14000 nucleotides, median = about 7600 nucleotides) (Table 2).

Finally, a small number of deHERV loci with the best discriminating power were selected, identifying as the final subset a total of 17 deHERV loci ordered on the basis of decreasing mean and variance values of expression, in order to select the most significant ones (Figure 8). In both cases, the 17 loci (13 of which shared between the two analyzes based on mean and variance of expression) make it possible to clearly distinguish MS patients from healthy controls (Figure 8, Table 2). The loci deHERV included in this subset have minimum levels of statistical significance between 7:08⁻¹⁴ and 0.005, and most of the loci is down-regulated in patients with MS (healthy controls: TPM values between 5.6 and 0.5, average = 2.1, median = 1.7; MS patients: TPM values between 2.9 and 0.4, mean = 1.16, median = 0.9) (Table 3).

**Table 2. Description of the set of deHERV loci discriminating for MS**

| **N°** | **HER V ID** | **Coordinates hg38 (chromosome: beginning-end)** | **st.** | **Bases** | **Group** | **DE in SM** | **Log2 Fold Chan ge** | **Padj** | **Averag e TPM (C)** | **Averag e TPM (SM)** |
|---|---|---|---|---|---|---|---|---|---|---|
| 1 | 673 | 2:127369875-127377853 | + | 7979 | HML5 | DOWN | -1.13 | 7.08E-14 | 5.57 | 2.89 |
| 2 | 4841 | 20:41594586-41602330 | + | 7745 | HERVIP | DOWN | -1.75 | 5.52E-09 | 1.21 | 0.42 |
| 3 | 4488 | 17:77167827-77175201 | - | 7375 | HERVIP | DOWN | -1.36 | 2.47E-08 | 2.06 | 0.93 |
| 4 | 2q13 | 2:112039346-112044989 | - | 5644 | HERVW | UP | 1.86 | 2.68E-08 | 0.45 | 1.84 |
| 5 | 857 | 3:5140823-5149818 | - | 8996 | HERVAD P | DOWN | -1.01 | 1.09E-07 | 2.04 | 1.14 |
| 6 | 2073 | 6:11103051-11112191 | - | 9141 | HERVFR D | DOWN | -1.31 | 1.27E-07 | 1.38 | 0.62 |
| 7 | 1080 | 3:119978070-119990309 | + | 12240 | LTR19 | DOWN | -1.15 | 7.11E-07 | 1.09 | 0.55 |
| 8 | 977 | 3:72023117-72028917 | + | 5801 | HERVH | DOWN | -1.33 | 3.41E-06 | 2.90 | 1.31 |
| 9 | 750 | 2:174202252-174216415 | + | 14164 | HUERSP 2 | DOWN | -1.20 | 2.24E-05 | 0.59 | 0.29 |
| 10 | 4153 | 14:53128745-53135507 | + | 6763 | HERVH | DOWN | -1.38 | 3.15E-05 | 2.90 | 1.26 |
| 11 | 1278 | 3:193599956-193613333 | - | 13378 | HEPSI1 | DOWN | -1.02 | 3.68E-05 | 1.35 | 0.76 |
| 12 | 6q21 a | 6:106228137-106235814 | + | 7678 | HERVW | DOWN | -1.23 | 4.84E-05 | 0.87 | 0.42 |
| 13 | 3222 | 10:35403060-35410120 | - | 7061 | HERVH | DOWN | -1.19 | 5.39E-05 | 5.23 | 2.54 |
| 14 | 5883 | 1:48437944-48446631 | + | 8688 | HERV9 | DOWN | -1.82 | 8.25E-05 | 0.47 | 0.14 |
| 15 | 3167 | 9:131680373-131686728 | + | 6356 | HML3 | DOWN | -1.14 | 0.0001 | 1.39 | 0.72 |
| 16 | 715 | 2:152701399-152709042 | + | 7644 | HERVIP | DOWN | -1.02 | 0.0001 | 1.28 | 0.72 |
| 17 | 5861 | 1:37907865-37914094 | + | 6230 | HERVH | DOWN | -1.02 | 0.0003 | 2.11 | 1.16 |
| 18 | 3123 | 9:100139459-100146544 | - | 7086 | HERVH | DOWN | -1.08 | 0.0003 | 1.66 | 0.88 |
| 19 | 2982 | 8:144858915-144868670 | - | 9756 | LTR46 | DOWN | -1.10 | 0.0004 | 0.95 | 0.50 |
| 20 | 3203 | 10:20118010-20124934 | - | 6925 | HERVH | DOWN | -1.11 | 0.0004 | 3.34 | 1.71 |
| 21 | 2196 | 6:70436656-70448522 | - | 11867 | HML3 | DOWN | -1.22 | 0.0004 | 0.54 | 0.27 |
| 22 | 1992 | 5:131571798-131582180 | + | 10383 | HERVH | DOWN | -1.12 | 0.0004 | 0.84 | 0.43 |
| 23 | 2307 | 6:118617102-118626810 | + | 9709 | HERV9 | DOWN | -1.23 | 0.0005 | 0.89 | 0.43 |
| 24 | 3678 | 12:1711131-1715463 | - | 4333 | HERV9 | DOWN | -1.20 | 0.0005 | 1.44 | 0.70 |
| 25 | 1081 | 3:120038402-120045736 | + | 7335 | HERVIP | DOWN | -1.26 | 0.0005 | 0.87 | 0.41 |
| 26 | 4779 | 19:53831404-53845084 | + | 13681 | HERVH | DOWN | -2.79 | 0.0006 | 0.24 | 0.04 |
| 27 | 1110 | 3:128828222-128834858 | - | 6637 | HERVH | DOWN | -1.20 | 0.0007 | 1.28 | 0.64 |
| 28 | 749 | 2:172542091-172548758 | + | 6668 | HERVH | DOWN | -1.79 | 0.0009 | 0.43 | 0.14 |
| 29 | 2014 | 5:147868278-147874526 | - | 6249 | HERVH | UP | 2.62 | 0.0009 | 0.11 | 0.73 |
| 30 | 3741 | 12:26777173-26782083 | + | 4911 | HERVL | DOWN | -1.55 | 0.0009 | 0.82 | 0.31 |
| 31 | 550 | 2:62088710-62100497 | - | 11788 | MER4 | DOWN | -1.21 | 0.0014 | 0.40 | 0.20 |
| 32 | 2059 | 6:2310409-2317792 | - | 7384 | Harlequin | DOWN | -1.27 | 0.0017 | 0.61 | 0.29 |
| 33 | 1756 | 5:10716140-10724937 | + | 8798 | HERVL | DOWN | -1.35 | 0.0017 | 0.44 | 0.20 |
| 34 | 1286 | 4:3046337-3056570 | - | 10234 | MER57 | DOWN | -1.74 | 0.0021 | 0.30 | 0.10 |
| 35 | 2126 | 6:38616529-38624444 | + | 7916 | HERV9 | DOWN | -1.22 | 0.0022 | 0.61 | 0.30 |
| 36 | 4097 | 14:30965849-30972548 | + | 6700 | HERVIP | DOWN | -1.10 | 0.0025 | 1.09 | 0.57 |
| 37 | 791 | 2:203960014-203967570 | - | 7557 | HERVH | DOWN | -2.06 | 0.0028 | 0.31 | 0.08 |
| 38 | 1109 | 3:128836995-128842027 | - | 5033 | HERVH | DOWN | -1.25 | 0.0037 | 0.81 | 0.38 |
| 39 | 4165 | 14:59610765-59618496 | + | 7732 | HUERSP 1 | DOWN | -1.08 | 0.0042 | 1.17 | 0.61 |
| 40 | 5972 | 1:94445506-94448856 | + | 3351 | HML2 | DOWN | -1.28 | 0.0064 | 1.06 | 0.49 |
| 41 | 4555 | 18:46868452-46877781 | + | 9330 | HERVIP | DOWN | -1.41 | 0.0068 | 0.38 | 0.16 |
| 42 | 3688 | 12:8319624-8324920 | - | 5297 | HERVT | DOWN | -1.03 | 0.0074 | 1.21 | 0.69 |
| 43 | 2721 | 8:8171833-8181319 | + | 9487 | HERVE | DOWN | -1.38 | 0.0080 | 0.30 | 0.13 |

| | | | | | | | | | | |
|---|---|---|---|---|---|---|---|---|---|---|
| *HERV ID from Vargiu et al. 2016 and Grandi et al. 2016 (HERV-W). St = strand, DE = differentially expressed (down-regulated or up-regulated in SM), padj = adjusted p-value (significance threshold = 0.01, the change in expression of the HERV loci was measured in log2 fold changes), TPM = transcripts per million kilobases, C = healthy controls, MS = patients with multiple sclerosis* | | | | | | | | | | |

**Table 3. Description of the selected subset of deHERV loci with discriminating power for MS**

| **N°** | **HER V ID** | **Coordinates hg38 (chromosome: beginning-end)** | **St** | **Bases** | **Grou p** | **DE in SM** | **Log2 Fold Chan ge** | **Padj** | **Mean TPM (HC)** | **Mean TPM (SM)** | **SEQ ID No.** |
|---|---|---|---|---|---|---|---|---|---|---|---|
| 1 | 673 | 2:127369875-127377853 | + | 7979 | HML5 | DOW N | -1.13 | 7.08E-14 | 5.57 | 2.89 | **SEQ ID No.1** |
| 3 | 4488 | 17:77167827-77175201 | - | 7375 | HER VIP | DOW N | -1.36 | 2.47E-08 | 2.06 | 0.93 | **SEQ ID No.2** |
| 4 | 2q13 | 2:112039346-112044989 | - | 5644 | HER VW | UP | 1.86 | 2.68E-08 | 0.45 | 1.84 | **SEQ ID No.3** |
| 5 | 857 | 3:5140823-5149818 | - | 8996 | HER VADP | DOW N | -1.01 | 1.09E-07 | 2.04 | 1.14 | **SEQ ID No.4** |
| 6 | 2073 | 6:11103051-11112191 | - | 9141 | HER VFRD | DOW N | -1.31 | 1.27E-07 | 1.38 | 0.62 | **SEQ ID No.5** |
| 7 | 1080 | 3:119978070-119990309 | + | 12240 | LTR1 9 | DOW N | -1.15 | 7.11E-07 | 1.09 | 0.55 | **SEQ ID No.6** |
| 8 | 977 | 3:72023117-72028917 | + | 5801 | HER VH | DOW N | -1.33 | 3.41E-06 | 2.90 | 1.31 | **SEQ ID No.7** |
| 10 | 4153 | 14:53128745-53135507 | + | 6763 | HER VH | DOW N | -1.38 | 3.15E-05 | 2.90 | 1.26 | **SEQ ID No.8** |
| 11 | 1278 | 3:193599956-193613333 | - | 13378 | HEPS I1 | DOW N | -1.02 | 3.68E-05 | 1.35 | 0.76 | **SEQ ID No.9** |
| 13 | 3222 | 10:35403060-35410120 | - | 7061 | HER VH | DOW N | -1.19 | 5.39E-05 | 5.23 | 2.54 | **SEQ ID No.10** |
| 17 | 5861 | 1:37907865-37914094 | + | 6230 | HER VH | DOW N | -1.02 | 0.0003 | 2.11 | 1.16 | **SEQ ID No.11** |
| 18 | 3123 | 9:100139459-100146544 | - | 7086 | HER VH | DOW N | -1.08 | 0.0003 | 1.66 | 0.88 | **SEQ ID No.12** |
| 20 | 3203 | 10:20118010-20124934 | - | 6925 | HER VH | DOW N | -1.11 | 0.0004 | 3.34 | 1.71 | **SEQ ID No.13** |
| 16 (m) | 715 | 2:152701399-152709042 | + | 7644 | HER VIP | DOW N | -1.02 | 0.0001 | 1.28 | 0.72 | **SEQ ID No.14** |
| 2(v) | 4841 | 20:41594586-41602330 | + | 7745 | HER VIP | DOW N | -1.75 | 5.52E-09 | 1.21 | 0.42 | **SEQ ID No.15** |
| 19 (m) | 2982 | 8:144858915-144868670 | - | 9756 | LTR4 6 | DOW N | -1.1 | 0.0004 | 0.95 | 0.50 | **SEQ ID No.16** |
| **23** (v) | 2307 | 6:118617102-118626810 | + | 9709 | HER V9 | DOW N | -1.23 | 0.0005 | 0.89 | 0.43 | **SEQ ID No.17** |

| | | | | | | | | | | | |
|---|---|---|---|---|---|---|---|---|---|---|---|
| *The loci with the words (m) and (v) have been identified on the basis of only the mean and variance of expression, respectively, while the others are common to the two selections. HERV ID from Vargiu et al. 2016 and Grandi et al. 2016 (HERV-W). St = strand, DE = differentially expressed (down-regulated or up-regulated in SM), padj = adjusted p-value (significance threshold = 0.01, the change in expression of the HERV loci was measured in log2 fold changes), TPM = transcripts per million kilobases, C = healthy controls, MS = patients with multiple sclerosis* | | | | | | | | | | | |

For this minimum discriminant subset of 17 deHERV loci, specific primer pairs were designed, suitable for verifying their expression levels by Real-Time PCR technique. This analysis confirmed the changes in expression detected through the bioinformatic analysis of the data obtained from the sequencing.

The analysis described above was developed starting from peripheral blood monocytes, but it can be used on RNA derived from any sample obtained from a subject, by way of non-limiting example RNA derived from tissues and body fluids such as blood, plasma, biopsy, muscle biopsy, spinal fluid, cerebrospinal fluid, saliva, oral mucosal cells.

The analysis and comparison of the expression levels can be carried out as described above or by means of techniques known to those skilled in the art that are currently available or that can be developed in the future; by way of non-limiting example, this analysis can be performed by RT-PCR, Real Time PCR, microarray, Northern Blotting, in situ hybridization, molecular beacons, forced intercalation probes and other RNA detection techniques known to those skilled in the art.

The method described above and the choice of data analysis deriving from sequencing of total messenger RNA with this method is particularly preferred as it is able to evaluate the expression of thousands of sequences with a high degree of structural homology, which makes them highly similar to each other. The preferred method is based on the mapping of all the transcripts to the sequence of the human genome, and the subsequent univocal identification based on their precise chromosomal position. In this way, "doubtful" transcripts as they map to several positions are excluded in the preliminary phase, and the subsequent quantifications concern only the sequences actually and univocally deriving from the HERV taken into consideration. The use of more traditional approaches is however possible by providing for the necessary passage, within the reach of experts, of designing specific probes or pairs of primers for each HERV sequence.

The object of the present invention is therefore a method for the in vitro diagnosis of multiple sclerosis comprising an analysis step of the expression levels of the HERV of the HERV of sequence SEQ ID No. 1 in a biological sample obtained from a subject and a step comparing said levels with the levels found in samples obtained from healthy individuals, in which decreased levels of expression by minus one of said transcripts indicate the presence of multiple sclerosis in the subject.

In particular, the subject of the present invention is an in vitro method for the diagnosis of multiple sclerosis in an individual, the method comprising the following basic steps:
- to isolate ex vivo an RNA sample from a biological sample obtained from a subject:
- determine the expression levels of the transcript of the HERV of sequence SEQ ID No. 1,
in which a decrease of the expression levels of said HERVs in the sample under examination, compared to the levels of the corresponding product in a control sample, is indicative of the presence of multiple sclerosis in the subject.

The object of the present invention is therefore a method for thediagnosis *in vitro* of multiple sclerosis in an individual in which the levels of expression analyzed is the HERV transcript SEQ ID No. 1.

The expression levels decreased by the HERV transcript of SEQ ID No 1 in the sample under examination compared to the levels detected in the sample obtained from a healthy individual are indicative of the presence of multiple sclerosis in the subject.

The object of the present invention is therefore a human endogenous retroviral sequence (HERV) selected and validated in a specific way among the tens of thousands of similar sequences present in the genome which has shown early diagnostic power for the pathology Multiple Sclerosis ( SM). More in detail, the association with the presence of multiple sclerosis is represented by the overall pattern of expression of the set of sequences indicated which, taken together, showed a significantly higher diagnostic-predictive power than the evaluation of a single sequence or a subset of them for this reason, also in order to reach the most sensitive method in terms of diagnostic reliability, it is preferable to consider for the purposes of the invention this set of specific sequences as a whole, meaning it as a single panel of biological indicators, rather than patenting each of the single sequences, since the diagnostic power observed on the basis of the expression of the 43 HERV sequences according to table 2 as a whole, and preferably on the basis of the minimum discriminant set of the 17 sequences reported in table 3, is undoubtedly more accurate than that which is would have considered the expression individually the single sequences.

Thus, the present invention relates to the use of the HERV of SEQ ID No 1 as biomarker in the in vitro diagnosis of multiple sclerosis.

With the findings of the invention, therefore, the need to identify markers strongly and uniquely correlated to the presence of MS pathology and the need for an analysis method capable of overcoming the technical problems related to the methods commonly used for the analysis of sequences are satisfied.

## Claims

1. An in vitro method for diagnosing multiple sclerosis in an individual, the method comprising the following basic steps:
- isolating an RNA sample ex vivo from a biological sample obtained from a subject;
- determining the expression levels of the human endogenous retrovirus (HERV) transcripts of sequence SEQ ID No. 1;
- compare these levels of expression with the levels of expression of the same HERV detected in a sample of RNA isolated ex vivo from a sample obtained from a healthy individual in which the decreased levels of expression of said HERV in the test sample compared to the levels detected in the sample obtained from a healthy individual are indicative of the presence of multiple sclerosis in the subject.

2. Method according to claim 1 wherein the analysis to determine the expression levels is carried out by deep sequencing, RT-PCR, Real Time PCR, microarray, Northern blotting, in situ hybridization, molecular beacons, or other forced intercalation probes RNA detection techniques, preferably RNAseq.

3. Method for the in vitro diagnosis of multiple sclerosis comprising the following basic steps:
- deep sequencing of polyadenylated RNA, isolated ex vivo, from a biological sample of a subject to generate sequencing reads,
- bioinformatic analysis carried out through a system of computer comprising:
I. To align the sequencing reads obtained with a reference human genome;
II. Determine the relative quantities of the sequence in the sample corresponding to the HERV specific transcripts of SEQ ID 1 ;
- compare said relative quantities with the relative quantities detected with said passages in a sample of polyadenylated RNA isolated ex vivo from a sample of a healthy individual in which the decreased expression levels of said HERV in the sample under examination compared to the levels detected in the sample obtained from a healthy individual are indicative of the presence of multiple sclerosis in the subject.

4. Method according to the preceding claims, wherein said samples are RNA samples derived from in-vitro tissues and body fluids, preferably blood, plasma, biopsy, muscle biopsy, spinal fluid, cerebrospinal fluid, saliva, oral mucosa cells.

5. Use of HERV of SEQ ID No 1 as biomarker in in vitro diagnosis of multiple sclerosis.

6. Use of the HERV of SEQ ID No 1 according to the preceding claim in which expression levels decreased of the HERV transcripts of SEQ ID No 1 compared to the levels detected in samples obtained from a healthy individual are indicative of the presence of multiple sclerosis in the subject.

## Patentansprüche

1. In-vitro-Verfahren zur Diagnose von Multipler Sklerose bei einem Individuum, das Verfahren umfassend die folgenden grundlegenden Schritte:
- Ex-vivo-Isolierung einer RNA-Probe aus einer biologischen Probe eines Probanden;
- Bestimmung der Expressionsniveaus von Transkripten des humanen endogenen Retrovirus (HERV) der Sequenz SEQ ID Nr. 1;
- Vergleich dieser Expressionsniveaus mit den Expressionsniveaus desselben HERV, die in einer ex vivo aus einer Probe eines gesunden Individuums isolierten RNA-Probe nachgewiesen wurden, wobei die reduzierten Expressionsniveaus des besagten HERV in der getesteten Probe im Vergleich zu den in der Probe eines gesunden Individuums nachgewiesenen Niveaus auf das Vorliegen von Multipler Sklerose bei dem Probanden hinweisen.

2. Verfahren nach Anspruch 1, wobei die Analyse zur Bestimmung der Expressionsniveaus mittels Deep Sequencing, RT-PCR, Real Time PCR, Microarray, Northern Blotting, In-situ-Hybridisierung, Molecular Beacons oder anderen RNA-Nachweisverfahren unter Verwendung von forcierten Interkalationssonden, vorzugsweise RNAseq, durchgeführt wird.

3. Verfahren zur In-vitro-Diagnose von Multipler Sklerose, das die folgenden grundlegenden Schritte umfasst:
- Tiefensequenzierung von ex vivo isolierter polyadenylierter RNA aus einer biologischen Probe eines Probanden zur Generierung von Sequenzierungs-Reads;
- bioinformatische Analyse mittels eines Computersystems, umfassend:
I. Abgleich der erhaltenen Sequenzierungs-Reads mit einem menschlichen Referenzgenom;
II. Bestimmung der relativen Mengen von Sequenzen in der Probe, die den HERV-spezifischen Transkripten der SEQ ID 1 entsprechen;
- Vergleich dieser relativen Mengen mit den relativen Mengen, die während der Passagen in einer Probe polyadenylierter RNA, die ex vivo aus einer Probe eines gesunden Probanden isoliert wurde, nachgewiesen wurden, wobei die verringerten Expressionsniveaus des besagten HERV in der untersuchten Probe im Vergleich zu den in der Probe eines gesunden Probanden nachgewiesenen Niveaus auf das Vorliegen von Multipler Sklerose bei dem Probanden hinweisen.

4. Verfahren gemäß den vorhergehenden Ansprüchen, wobei es sich bei den Proben um RNA-Proben aus in vitro freigesetzten Geweben und Körperflüssigkeiten handelt, vorzugsweise aus Blut, Plasma, Biopsie, Muskelbiopsie, Rückenmarksflüssigkeit, Zerebrospinalflüssigkeit, Speichel oder Mundschleimhautzellen.

5. Verwendung der HERVs der SEQ ID Nr. 1 als Biomarker in der In-vitro-Diagnose von Multipler Sklerose.

6. Verwendung der HERVs der SEQ ID Nr. 1 gemäß dem vorhergehenden Anspruch, wobei verringerte Expressionsniveaus der HERV-Transkripte der SEQ ID Nr. 1 im Vergleich zu den in Proben eines gesunden Individuums nachgewiesenen Niveaus auf das Vorliegen von Multipler Sklerose bei dem Subjekt hinweisen.

## Revendications

1. Méthode in vitro de diagnostic de la sclérose en plaques chez un individu, la méthode comprenant les étapes de base suivantes:
- isolement ex vivo d'un échantillon d'ARN à partir d'un échantillon biologique obtenu d'un sujet;
- détermination des niveaux d'expression des transcrits du rétrovirus humain endogène (HERV) de séquence SEQ ID n°1;
- comparer ces niveaux d'expression avec les niveaux d'expression du même HERV détectés dans un échantillon d'ARN isolé ex vivo d'un échantillon obtenu d'un individu sain, dans lequel les niveaux d'expression réduits dudit HERV dans l'échantillon testé, par rapport aux niveaux détectés dans l'échantillon obtenu d'un individu sain, sont indicatifs de la présence de sclérose en plaques chez le sujet.

2. Méthode selon la revendication 1, dans laquelle l'analyse visant à déterminer les niveaux d'expression est réalisée par séquençage profond, RT-PCR, PCR en temps réel, microarray, Northern blotting, hybridation in situ, balises moléculaires ou autres techniques de détection d'ARN par sondes à intercalation forcée, de préférence RNAseq.

3. Méthode de diagnostic in vitro de sclérose en plaques comprenant les étapes fondamentales suivantes:
- séquençage profond d'ARN polyadénylé, isolé ex vivo, à partir d'un échantillon biologique d'un sujet afin de générer des lectures de séquençage;
- analyse bioinformatique réalisée par un système informatique comprenant:
I. Aligner les lectures de séquençage obtenues avec un génome humain de référence;
II. Déterminer les quantités relatives des séquences dans l'échantillon correspondant aux transcrits spécifiques du HERV de SEQ ID 1;
- comparer lesdites quantités relatives aux quantités relatives détectées lors desdits passages dans un échantillon d'ARN polyadénylé isolé ex vivo d'un échantillon d'un individu sain, dans laquelle les niveaux d'expression réduits dudit HERV dans l'échantillon examiné, par rapport aux niveaux détectés dans l'échantillon obtenu d'un individu sain, sont indicatifs de la présence de sclérose en plaques chez le sujet.

4. Méthode selon les revendications précédentes, dans laquelle lesdits échantillons sont des échantillons ARN dérivés des tissus in vitro émis et de liquides corporels, de préférence de sang, de plasma, de biopsie, de biopsie musculaire, de liquide rachidien, de liquide céphalorachidien, de salive ou de cellules de la muqueuse buccale.

5. Utilisation des HERV de SEQ ID n° 1 comme biomarqueurs dans le diagnostic in vitro de la sclérose en plaques.

6. Utilisation des HERV de SEQ ID No 1 selon la revendication précédente, dans laquelle des niveaux d'expression diminués des transcrits HERV de SEQ ID No 1, comparés aux niveaux détectés dans des échantillons obtenus à partir d'un individu sain, sont indicatifs de la présence de sclérose en plaques chez le sujet.
